Europäisches Patentamt

European Patent Office

Office européen des brevets

(11) Publication number : **0 508 939 A2**

## (12) EUROPEAN PATENT APPLICATION

(21) Application number : **92670002.2**

(22) Date of filing : **18.03.92**

(51) Int. Cl.$^5$ : **A63F 9/22,** H04N 5/44

(30) Priority : **08.04.91 PT 97283**

(43) Date of publication of application :
**14.10.92 Bulletin 92/42**

(84) Designated Contracting States :
**DE FR IT PT**

(71) Applicant : **Gomes dos Santos, Alcino**
**Rua das Janelas Verdes, 1-1o**
**P-1200 Lisboa (PT)**

(72) Inventor : **Gomes dos Santos, Alcino**
**Rua das Janelas Verdes, 1-1o**
**P-1200 Lisboa (PT)**

(54) **Remotely controlled device for supplying scents and for creating ambiences.**

(57) The present system consists of complementary electric equipment to television and video, that will re-create scenic ambients, after the reception and decodification of the composed signal (4), in the separator/decodifier of signals (1), and posterior action of the decodified signal (6) in the actuating units (2) and (3).

The composed signal (4) containing information previously codified, is decodified in the separator/decodifier (1), of conception acording to current principles. They result from this decodification : signal of video (5) and the signals (electric levels) (6), that will actuate in the gears that equip the actuating units (2) and(3) and the exhaust (7).

The actuating unit (2) is composed basically by electrovalves (2.1), that will do to liberate the aromas contained in the respective capsules (2.2).

The actuating (3) is composed by generating equipments of wind(3.1), cold (3.2) and heat (3.3), of current conception, whose commands are analogous to the actuating unit (2).

Principal utilization --- re-creation of determined scenic ambients, through the smelling component, as well as the sensations of wind, cold, heat, etc..

The invention refers to a system that pretends to create a complement scenic for television and video, that re-creates more faithfully determined ambients, through in the smelling component, as well as from other type of sensations, as: wind, cold, heat, etc..

The originality of the proposed subject is founded on the fact from the re-creations may to be done at distance and for domestic use.

The present system bases by the reception of different information in a composed signal that, when separated and decodified by the separator/decodifier (1), will do to activate by electromecanical way, a gear of aspersion, reported as actuating unit (2), that contains an ensamble of aromas, or functioning with generating equipments of other efects: wind, cold, heat, etc., in the secondary acttuating unit (3).

The invention alludes to only the creation of efects, downstream of the succession of emissions, presupposing the possibility to program/codify upstream, that is, near the emission.

The command of actuating units (2) and (3) and of the exhaust (7) will be done by action of the signals (electric levels ) (6) after the partition and decodification of the composed signal (4), separating in to the video signal (5).

The composed signal (4), having been codified previously, is processed in the separator/decodifier (1), of conception according to current principles. From this separation/decodification result the separate signals: signal of video (5) and the signals ( electric levels ) (6).

For example, we describe a possible appliance of the insertion of the codified signal: the signals of code that will to integrate/the composed signal, in complement with the signals of video, would be inserted in the no visible lines of video, during the phase of codification.

The selection and actuation of determined efect will be done by the sending to the cited actuating units (2) and (3) of the corresponding signal (6).

The suspension of a determined aroma will be accelerated with the help of an exhaust (7) commanded by the system, through the corresponding signal(6), being neutralized the residual with a superposition of an ambient aroma, for small space of time, or the next situation aroma.

The actuating unit (2) is composed basically by pressurized capsules containing aromas (2.2), by deed of electrovalves (2.1) that will do to liberate the contained aromas.

The capsules will be placed in the gear of aspersion, reported as actuating unit (2), according to a reference of predetermined position, the gears of aspersion will may to be of several capacities and, so, to contain since sundry tens to sundry hundreds of capsules of aromas, these being elaborated beforehand, there being, still, the possibility to get others by synthetizing from the base aromas.

The actuating unit (2) will have a grate, conceived in order to constitute alveoles that individualize the going out of the aromas. The alveoles are destined to avoid that the aromas will be mixed with the residual of other aroma, adhered to the grate.

The secondary actuating unit (3) is composed by generating equipments of wind (3.1), cold (3.2) and heat (3.3), of current conception, commanded in a analogous way at the actuating unit (2).

The sensations of wind, cold, heat, etc. will be created, doing the signal (6) to start conventional generating souces of these, in the secondary actuating unit (3). For a larger fidelity of the sensation of heat or cold, without to perturb the room temperature, these will be generated to antechambers that will liberate, in the nick of time, in the form of wave of cold or wave of heat, with the help of a ventilator working by very small velocity.

The generator of heat (3.3) will have the antechamber with reflecting surface, for, by irradiation, to direct better the wave of heat.

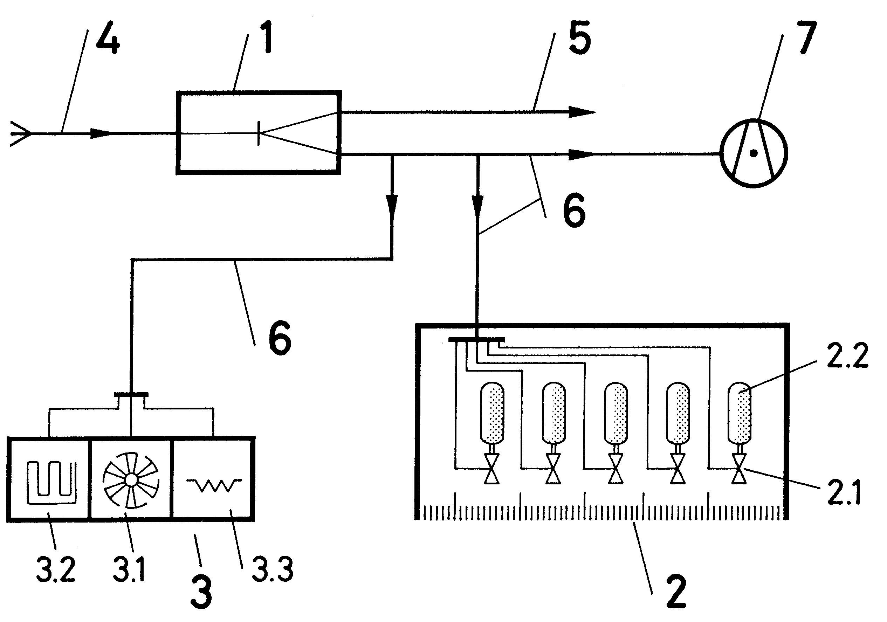

## Claims

1 - "SCENIC AMBIENTS RE-CREATOR" is characterized as being composed of a signal separator/decodifier (1), an actuating unit (2), and a secondary actuating unit (3).

2 - "SCENIC AMBIENTS RE-CREATOR", in accordance with claim No.1, is charaterized by the separator/decodifier (1) converting the information that integrates the signal recieved by a television receiver or read by video machine into electrical impulses that will activate the actuating units (2) or (3).

3 - "SCENIC AMBIENTS RE-CREATOR", in accordance with claims Nos.1 and 2, is characterized by the actuating unit (2) being constituted of a system that, by electronic command, functioning with different valves (2.1), will free corresponding aromas contained in small pressurized deposits (2.2).

4 - "SCENIC AMBIENTS RE-CREATOR", in accordance with claims Nos.1 and 2, is characterized by the secondary actuating unit (3), according to the identical principle of actuating unit (2), functioning with autonomous generating units of other efects, such as: cold(3.1) wind (3.2), heat (3.3), etc..

5 - "SCENIC AMBIENTS RE-CREATOR", in accordance with claims Nos.1, 2, 3 and 4, is characterized by the re-creations being made away from and toward the common utilizer.

6 - "SCENIC AMBIENTS RE-CREATOR", in accordance with claims Nos.1, 2, 3 and 4, is characterized by the re-creations being made from information contained in video recording supports, for video or television system users.